# EUROPÄISCHE PATENTSCHRIFT

(11) **EP 0 983 103 B1**
(45) Veröffentlichungstag und Bekanntmachung des Hinweises auf die Patenterteilung: **24.04.2002**
(21) Anmeldenummer: 98925557.5
(22) Anmeldetag: 08.05.1998
(51) Int. Cl.: A61M 15/00, A61M 11/02

(54) **VORRICHTUNG ZUR APPLIKATION EINES MEDIKAMENT-AEROSOLS ÜBER DIE LUNGE**
DEVICE FOR ADMINISTERING A MEDICINAL AEROSOL VIA THE LUNGS
DISPOSITIF POUR L'ADMINISTRATION D'UN AEROSOL MEDICAMENTEUX PAR LES POUMONS

(30) Priorität: 16.05.1997 DE 19720701
(43) Veröffentlichungstag der Anmeldung: 08.03.2000
(73) Patentinhaber: GSF-Forschungszentrum für Umwelt und Gesundheit GmbH, 85764 Oberschleissheim (DE)
(72) Erfinder: BRAND, Peter, D-82131 Gauting (DE); SELZER, Titus, D-80686 München (DE); SCHULZ, Holger, D-86899 Landsberg (DE); ROTH, Christa, D-65760 Eschborn (DE); HEYDER, Joachim, D-80802 München (DE)
(74) Vertreter: Reinhard - Skuhra - Weise & Partner
(86) Internationale Anmeldenummer: EP9802703
(87) Internationale Veröffentlichungsnummer: WO9852633

(56) Entgegenhaltungen:
- DE-A1- 2 809 255
- DE-A1- 4 300 880
- FR-A1- 2 604 093

## Beschreibung

Die Erfindung betrifft eine Vorrichtung zur Applikation eines Medikaments über die Lunge gemäß dem Oberbegriff des Patentanspruchs 1.

Die Verwendung des Inhalationsweges für die Applikation von Medikamenten gewinnt zunehmend an Bedeutung. Dabei werden außer dem Einsatz neuer lokal wirkender Medikamente für die Therapie von Lungenerkrankungen auch neue Therapiestrategien entwickelt, die die Lunge als Eingangsorgan für systemisch wirksame Substanzen verwenden.

Für die Verwendung des Inhalationsweges zur Applikation von Medikamenten werden wachsende Anforderungen an die Qualität des Inhalationsvorganges gestellt, die von den derzeit auf dem Markt befindlichen Geräten nicht erfüllt werden können. So ist es beispielsweise erforderlich, daß die vom Arzt verordnete Medikamentendosis möglichst genau und reproduzierbar appliziert wird, wobei die verabreichte Dosis und ihre Reproduzierbarkeit im wesentlichen von dem sogenannten Atemmanöver abhängt. Unter Atemmanöver ist das inhalierte Aerosolvolumen und der Atemfluss zu subsumieren, mit dem ein Patient die Inhalation durchführt. Durch geeignete Wahl von Atemmanöver und Vernebler bzw. durch Abstimmung des verwendeten Atemmanövers auf den verwendeten Vernebler (Teilchengröße) könnte der Depositionsort des Medikaments in der Lunge gezielt beeinflusst und damit eine vorwiegend zentrale oder vorwiegend periphere Pharmakonapplikation hervorgerufen werden.

Eine Vorrichtung der eingangs genannten Gattung ist bereits in der FR-A-2604093 offenbart. Als nachteilig ist dabei jedoch abzusehen, daß einerseits die Handhabung aufwendig ist und andererseits eine vom individuellen Atemmanöver abhängige Applikation nicht oder nur unzureichend durchzuführen ist.

Da sich im klinischen Alltag das von Patienten verwendete Atemmanöver üblicherweise nur wenig beeinflussen läßt bzw. das tatsächlich durchgeführte Atemmanöver sich fast gänzlich der ärztlichen Kontrolle entzieht, wäre eine exakte Vorgabe von Atemvolumen und Atemfluß bei der Inhalation therapeutischer Aerosole wünschenswert, da damit eine erhebliche Verbesserung der Inhalationstherapie und ihrer Reproduzierbarkeit verbunden sein würde. Außerdem ließe sich folglich bei exakter Einhaltung eines bestimmten Atemmanövers eine genauere Abschätzung der applizierten Dosis vornehmen und damit zugleich auch eine Kostenersparnis bei der Verwendung insbesondere teuerer Pharmaka erzielen.

Der Erfindung liegt demgemäß die Aufgabe zugrunde, die aufgezeigten Bedürfnisse durch eine funktionell einfach konzipierte Vorrichtung zu befriedigen.

Erfindungsgemäß wird diese Aufgabe durch die im Patentanspruch 1 genannten Merkmale gelöst. Bevorzugte Merkmale, die die Erfindung vorteilhaft weiterbilden, sind den nachgeordneten Patentansprüchen zu entnehmen.

Die erfindungsgemäße Vorrichtung besteht aus einem Inhalationsmundstück, mit einem zugeordneten einstellbaren Vernebler und aus einem Druckluft-Steuerventil, durch das ein voreinstellbarer Volumenstrom von Druckluft, die während einer einstellbaren Zeitdauer an den das flüssige Medikament enthaltenden Vernebler lieferbar ist, wobei zur Triggerung des Verneblungsbeginns des Verneblers ein Drucksensor vorgesehen ist.

Durch die erfindungsgemäße Vorrichtung wird es dem Patienten daher mit einfachen Mitteln ermöglicht, ein exakt vorgegebenes Pharmakonvolumen mit exakt vorgegebenen Atemfluß zu inhalieren. Dabei wird in günstiger Weise die im klinischen

Alltag für Therapie-Vernebler bereits vorgesehene separate Luftzuführung derart gesteuert, daß nur ein vorgegebenes Luftvolumen mit einem vorgegebenen Fluß an den Vernebler abgegeben wird. Nach der Erfindung wird somit die Luft des Verneblers auf eine vorwählbare Volumenstromstärke eingestellt, die vom Patienten nicht beeinflußt werden kann, wodurch sich eine Flußkonstanz ergibt. Der Zuluftvolumenstrom wird dann bevorzugt von einer elektronischen Steuerungseinheit über einen fest vorgegebenen Zeitraum eingeschaltet, um so ein exakt vorgegebenes Luftvolumen zu applizieren. Vorteilhaft wird durch die nach einer besonderen Ausgestaltung der Erfindung festlegbare Länge einer Atempause die Gesamtdeposition des Medikamentes erhöht und damit die interindividuelle Variabilität der deponierten Menge reduziert.

Für den praktischen Gebrauch der Vorrichtung ist es weiterhin günstig, wenn zur Triggerung des Vernebelungsbeginns des Verneblers ein auf Saugdruck im Inhalationsmundstück ansprechender Druckmesser vorgesehen ist.

Gemäß einer bevorzugten Ausgestaltung ist vor dem einstellbaren Vernebler ein Regler für das Konstanthalten des Volumenstroms angeordnet, wobei bevorzugt zur Kontrolle eines vorbestimmten Volumenstroms dem Regler ein Durchflußmesser nachgeordnet ist, welcher in einer speziellen Ausführungsform einen Schwebekörper aufweist. Diese Maßnahmen tragen zur Sicherung einer kontrollierten Inhalation therapeutischer Aerosole durch die Vorrichtung in vorteilhafter Weise bei.

Gemäß einer bevorzugten Ausgestaltung der Erfindung weist die Vorrichtung eine elektrische Steuerungseinheit für ihren Betrieb auf, wobei an der Steuerungseinheit eine Verneblungszeit und eine Pausenzeit einstellbar sind und wobei bevorzugt die elektronische Steuerungseinheit ein optisches und/oder akustisches Pausensignal für die Pause zwischen Inhalation und Exhalation aufweist, vorzugsweise in Form einer Leuchtdiode.

In günstiger Weise ist weiterhin die Zahl der Verneblungsperioden als Zahl von Atemzügen einstellbar.

Hinsichtlich der Ausbildung des Verneblers ist entweder eine Ausgestaltung als Düsenvernebler mit einer separaten Druckluftversorgung vorgesehen, die mittels des Druckluftsteuerventils ein- und ausschaltbar ist, wobei die separate Druckluftversorgung für den Düsenvernebler bevorzugt zwischen dem Druckluftsteuerventil und dem Durchflußmesser abzweigt.

Alternativ kann der Vernebler als Ultraschallwandler ausgebildet sein, dessen Hochfrequenzsignale direkt durch die elektronische Steuerungseinheit steuerbar sind.

Das Druckluftsteuerventil ist bevorzugt als Magnetventil ausgebildet, und vor dem Steuerventil sind zur Anpassung an die individuellen Druckluftverhältnisse ein Druckminderer und ein Manometer angeordnet.

Vorteilhaft kann gemäß der Erfindung durch die gleichzeitige Wahl von Inhalationsfluß und inhalierten Volumen ein breites Spektrum von Atemmanövern der medizinischen Anwendung erschlossen werden, wobei über unterschiedliche Atemmanöver der Depositionsort in der Lunge in gewünschter Weise beeinflußbar ist. Damit ist es erstmals möglich, Medikamente, die beispielsweise in zentralen Lungenbereichen wirken sollen, überwiegend auch dort zu deponieren.

Ein weiterer Vorteil der erfindungsgemäßen Vorrichtung besteht darin, daß damit eine routinemäßige Durchführung von Inhalationen bei Patienten im klinischen Bereich verbessert werden kann, weil ein Patient durch die vorgesehene Atemtriggerung der Inhalation keine Probleme hinsichtlich der Synchronisierung des Starts des Verneblers und des Beginns der Inhalation hat. Außerdem können vorteilhaft Fehler bei der Inhalation, die auf eine unsachgemäße Handhabung des Verneblers zurückzuführen sind, deutlich vermindert werden.

Nachfolgend wird die Erfindung unter Bezugnahme auf die beigefügten Zeichnungen näher erläutert. Es zeigen:
- Fig. 1: eine schematisierte Darstellung eines Ausführungsbeispiels einer erfindungsgemäßen Vorrichtung; und
- Fig.2: eine schematisierte Komponentenzusammenstellung des Ausführungsbeispiels der Erfindung gemäß Fig.1.

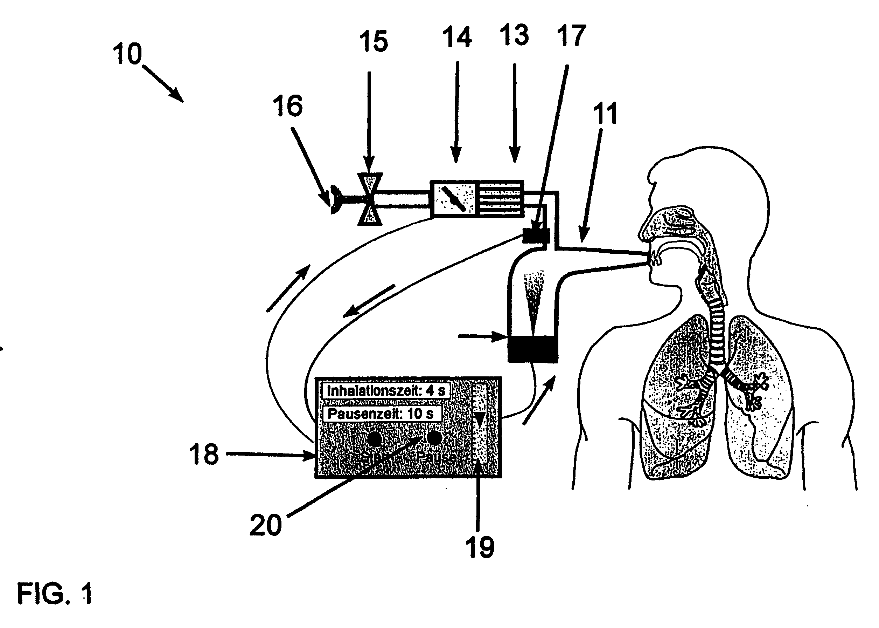

In Figur 1 ist an einem Ausführungsbeispiel schematisiert das Zusammenwirken einzelner Komponenten einer erfindungsgemäßen Vorrichtung 10 dargestellt. Die Vorrichtung 10 zur Applikation eines Medikament-Aerosols über die Lunge besteht aus einem Inhalationsmundstück 11 mit einem zugeordneten in seinen Betriebsphasen und Intensität/Frequenz einstellbaren Vernebler 12. In Leitungsverbindung mit dem Inhalationsmundstück 11 befinden sich ein Volumenstromregler 13, ein Druckluftsteuerventil 14, das bevorzugt als Magnetventil ausgebildet ist, ein Druckminderer 15 und ein Drucklufteingang 16. Mit 17 ist ein Drucksensor bezeichnet, der zur Triggerung des Verneblungsbeginns des Verneblers 12 auf Saugdruck im Mundstück anspricht.

Eine elektronische Steuerungseinheit 18 ist funktionell mit dem Druckluftsteuerventil 14, dem Drucksensor 17 und dem Vernebler 12 verbunden. Die elektronische Steuerungseinheit 18 ist schematisiert als Gehäuseblock dargestellt, das zugleich eine optische Anzeige eines Durchflußmessers 19 zur Kontrolle des Inhalationsflusses, beispielsweise mit einem Wertebereich von 0 bis 1000 cm³/s besitzt. Der Volumenstromregler 13 dient zum konstant halten des Inhalationsflusses in einem Wertebereich von beispielsweise 0 - 1000 cm³/s. Das Druckluftsteuerventil 14 ist bevorzugt als Magnetventil ausgebildet, das die Luftzufuhr schaltet.

Bei der elektronischen Steuerungseinheit 18 sind weiterhin in nicht dargestellter Weise die Inhalationszeit, die Pausenzeit und die Zahl der Atemzüge einstellbar, wobei als Pausensignal eine Leuchtdiode 20 vorgesehen ist.

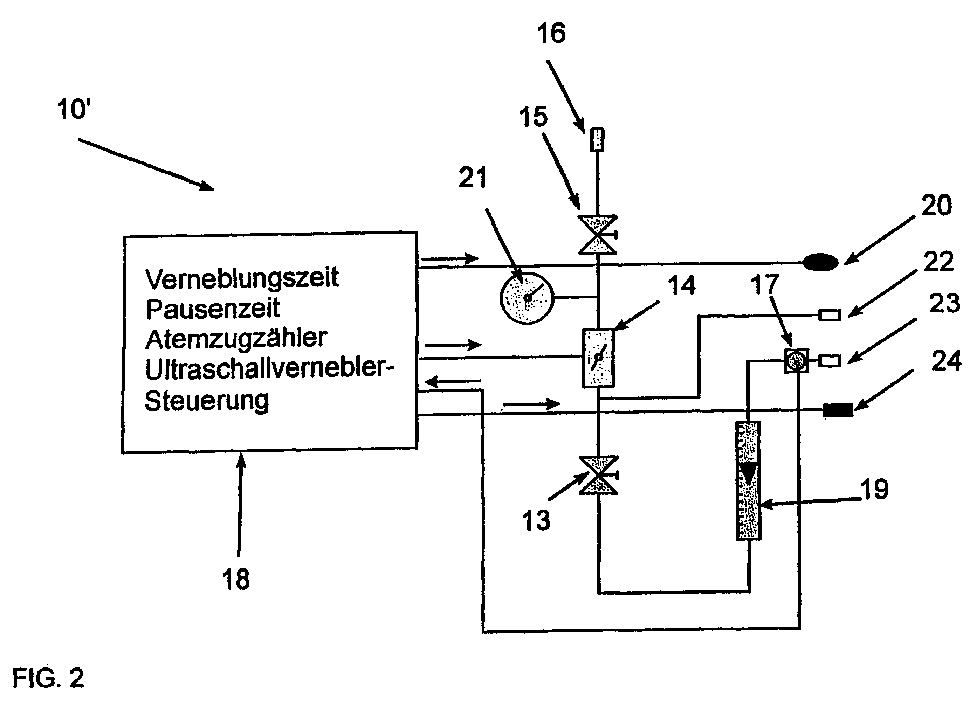

Ergänzend zu dem Ausführungsbeispiel gemäß Figur 1 ist schematisiert in Figur 2 ein Manometer 21 zwischen dem Druckminderer 15 und dem Magnetventil 14 zur Einstellung des Betriebsdrucks des Verneblers, beispielsweise in einem Wertebereich von 0 - 2 bar, vorgesehen. Außerdem ist bei dieser Ausführungsform eine selbstabstellende Druckluftkupplung 22 zur Düsenversorgung eines Verneblers, in Ausbildung als Düsenvernebler vorgesehen. Zum Anschließen des Verneblers 12 selbst ist bei 23 ein Adapter dargestellt, während mit 24 eine Signalleitung zum Schalten eines als Ultraschallvernebler ausgebildeten Verneblers angegeben ist.

In dem Block der elektronischen Steuerungseinheit 18 sind textlich die einzelnen einstellbaren Funktionen wie Verneblungszeit bzw. Inhalationszeit, die Pausenzeit und die Ultraschallverneblersteuerung erwähnt, wobei zur Erfassung der Atemzüge ein Atemzugzähler angesprochen ist.

Zur Verwendung der erfindungsgemäßen Vorrichtung 10 wird zunächst der Sollfluß der Inhalation mit dem Schwebkörper-Flußmesser 19 kontrolliert und an dem Flußkonstanzhalter bzw. Volumenstromregler 13 auf den gewünschten Betrag eingestellt. Anschließend wird die gewünschte Inhalationszeit über nicht dargestellte Taster an der elektronischen Steuerungseinheit 18 eingestellt, beispielsweise in einem Bereich von 0 - 20 Sekunden. Aus der Inhalationszeit und dem Inhalationsfluß ergibt sich dann das inhalierte Volumen. Die gewünschte Pausenzeitdauer wird über nicht dargestellte Taster ebenfalls an der Steuerungseinheit 18 eingestellt, beispielsweise in einem Bereich von 0 - 20 Sekunden. Außerdem wird der Atemzugzähler auf Null gesetzt.

Nach dieser Vorbereitung kann nun die Inhalation in der Form durchgeführt werden, daß der Patient am Mundstück 11 saugt, wodurch der Drucksensor 17 anspricht und automatisch die Inhalation startet. Für die Dauer der vorgewählten Inhalationszeit wird der Vernebler 12 mit Druckluft versorgt, und aus dem Mundstück 11 tritt mit vorgewählten Fluß das gewünschte Medikament in Aerosolform. Nach Ablauf der Inhalationszeit wird die Druckluftzufuhr unterbrochen und der Patient kann nicht weiter inhalieren. Die Leuchtdiode 20 signalisiert dem Patienten, den Atem anzuhalten. Wenn die Pausenzeit abgelaufen ist, erlischt die Pausenzeit-Leuchtdiode 20, der Patient atmet aus und der Atemzugzähler wird inkrementiert. Die Vorrichtung ist nun bereit zur nächsten Inhalation.

Eine weitere Einstellmöglichkeit für die Vernebelung besteht bei einem Düsenvernebler darin, die zwischen dem Magnetventil 14 und dem Durchflußstromregler 13 abgezweigte Druckluftversorgung zu steuern. Bei Verwendung eines Ultraschallverneblers kann das erforderliche Hochfrequenzsignal entsprechend seperat gesteuert werden.

## Patentansprüche

1. Vorrichtung zur Applikation eines Medikaments über die Lunge, mit
einem Inhalationsmundstück (11)
einem dem Inhalationsmundstück (11) zugeordneten einstellbaren Vernebler (12), der das Medikament in flüssiger Form enthält, und mit
einem Druckluftsteuerventil (14), durch das ein mittels eines Reglers (13) voreinstellbarer Druckluft-Volumenstrom während einer einstellbaren Zeitdauer an den Vernebler (12) lieferbar ist,
**dadurch gekennzeichnet,**
**daß** zur Triggerung des Verneblungsbeginns des Verneblers (12) ein Drucksensor (17) vorgesehen ist.

2. Vorrichtung nach Anspruch 1,
**dadurch gekennzeichnet,**
**daß** der zur Triggerung des Verneblungsbeginns des Verneblers (12) vorgesehene Drucksensor ein auf Saugdruck im Mundstück (11) ansprechender Drucksensor (17) ist.

3. Vorrichtung nach Anspruch 1 oder 2,
**dadurch gekennzeichnet,**
**daß** der Regler (13) für das Konstanthalten des Volumenstroms vor dem zeitlich einstellbaren Vernebler (12) angeordnet ist.

4. Vorrichtung nach Anspruch 3,
**dadurch gekennzeichnet,**
**daß** zur Kontrolle eines vorbestimmten Volumenstroms dem Regler (13) ein Durchflußmesser (19) nachgeordnet ist.

5. Vorrichtung nach Anspruch 4,
**dadurch gekennzeichnet,**
**daß** der Durchflußmesser (19) einen Schwebekörper aufweist.

## Claims

1. Device for administration of a medicament via the lungs, comprising
an inhalation mouthpiece (11),
an adjustable vaporiser (12) associated to said inhalation mouthpiece (11), which vaporiser contains said medicament in a liquid form, and comprising
a compressed-air control valve (14) through which a volumetric flow of compressed air, which can be preset by means of a controller (13), can be delivered to said vaporiser (12) throughout a settable period of time,
**characterised in**
**that** a pressure sensor (17) is provided for triggering the beginning of the vaporising operation of said vaporiser (12).

2. Device according to Claim 1,
**characterised in**
**that** said pressure sensor (17) provided for triggering the beginning of the vaporising operation of said vaporiser (12) is a pressure sensor (17) responsive to a suction pressure in said mouthpiece (11).

3. Device according to Claim 1 or 2,
**characterised in**
**that** said controller (13) is disposed upstream of said vaporiser (12) which is adapted to be set in terms of time, for maintaining the volumetric flow constant.

4. Device according to Claim 3,
**characterised in**
**that** a flow meter (19) is disposed downstream of said controller (13) for control of a predetermined volumetric flow.

5. Device according to Claim 4,
**characterised in**
**that** said flow meter (19) includes a floating body.

## Revendications

1. Dispositif destiné à administrer un médicament dans le poumon, comprenant :
un bec d'inhalation (11),
un nébuliseur (12) réglable, associé au bec d'inhalation (11), lequel nébuliseur contient le médicament sous forme liquide, et
une vanne de commande d'air comprimé (14), qui permet de fournir un débit d'air comprimé préréglable au moyen d'un régulateur (13) pendant un intervalle de temps réglable sur le nébuliseur (12),
**caractérisé en ce**
**qu'**il est prévu un capteur de pression (17) destiné à déclencher le début du processus de nébulisation sur le nébuliseur (12).

2. Dispositif selon la revendication 1, **caractérisé en ce que** le capteur de pression prévu pour déclencher le début du processus de nébulisation sur le nébuliseur (12) est un capteur de pression (17) activable par la pression d'aspiration dans le bec d'inhalation (11).

3. Dispositif selon la revendication 1 ou 2, **caractérisé en ce que** le régulateur (13) destiné à maintenir constant le débit est monté en amont du nébuliseur (12) réglable dans le temps.

4. Dispositif selon la revendication 3, **caractérisé en ce qu'**un débitmètre (19) est monté en aval du régulateur (13) destiné à contrôler un débit prédéfini.

5. Dispositif selon la revendication 4, **caractérisé en ce que** le débitmètre (19) est muni d'un flotteur.
